# EUROPÄISCHE PATENTSCHRIFT

(11) **EP 3 101 167 B1**
(45) Veröffentlichungstag und Bekanntmachung des Hinweises auf die Patenterteilung: **29.01.2020**
(21) Anmeldenummer: 16001008.8
(22) Anmeldetag: 04.05.2016
(51) Int. Cl.: D06F 39/00, G01N 21/94, G01N 21/898, D06F 95/00

(54) **VERFAHREN ZUR PRÜFUNG GEWASCHENER ODER GEREINIGTER WÄSCHESTÜCKE**
METHOD FOR TESTING WASHED OR CLEANED LAUNDRY
PROCEDE DE VERIFICATION DE LINGES LAVES OU NETTOYES

(30) Priorität: 01.06.2015 DE 102015006765
(43) Veröffentlichungstag der Anmeldung: 07.12.2016
(60) Teilanmeldung: 19212233.1
(73) Patentinhaber: Herbert Kannegiesser GmbH, 32602 Vlotho (DE)
(72) Erfinder: Heinz, Engelbert, 32602 Vlotho (DE); Bringewatt, Wilhelm, 32457 Porta Westfalica (DE)
(74) Vertreter: Möller, Friedrich

(56) Entgegenhaltungen:
- EP-A1- 2 573 550
- EP-A2- 0 052 813
- WO-A1-96/09533
- WO-A1-2010/024082
- DE-A1- 3 242 447
- DE-A1- 19 624 905
- DE-U1- 20 105 840
- JP-A- H07 174 714
- US-A- 4 952 062
- US-A- 5 130 559

## Beschreibung

Die Erfindung betrifft ein Verfahren zur Prüfung gewaschener oder gereinigter Wäschestücke gemäß dem Oberbegriff des Anspruchs 1.

Bei der Reinigung bzw. beim Waschen von Wäschestücken aller Art, vor allem Flachwäsche und Formteile (z. B. Bekleidungsstücke), kann es vorkommen, dass nicht alle Flecken aus den verschmutzten Wäschestücken entfernt werden konnten, Löcher vorhanden sind und/oder die Wäschestücke noch andere Verunreinigungen oder Anhaftungen, wie vor allem Haare oder Fasern, aufweisen. Vor allem an den gewaschenen bzw. gereinigten Wäschestücken anhaftende Haare sind unakzeptabel. Solche Wäschestücke können nicht an Kunden zurückgeliefert werden.

Entscheidend ist auch insbesondere bei sogenannter Weißwäsche, vor allem wenn es sich dabei um Tischwäsche handelt, der Weißegrad.

Es ist aus der EP 2 573 550 A1 bekannt, Verunreinigungen, wie insbesondere Flecken und auch Risse durch eine Kamera auf einer Seite oder auch mehrere Kameras auf gegenüberliegenden oder der gleichen Seite der Wäschestücke zu ermitteln. Das geschieht, um getrennte Prüfungen der Wäschestücke, um auf beiden Seiten Verunreinigungen einerseits und Rissen andererseits feststellen zu können. Es ist aus dieser Schrift aber nicht bekannt, die unten liegende Nutz- oder auch Finishseite der Wäschestücke zu prüfen.

Die US 4 952 062 A befasst sich mit der Ermittlung von Fehlern in einem Gewebe. Dabei ist einer Seite des Gewebes eine Lichtquelle zugeordnet, während sich auf der gegenüberliegenden Seite des Gewebes ein Raster optischer Sensoren zur Ermittlung von Fehlern im zu inspizierenden Gewebe befindet. Mit der Prüfung der Finishseite gemangelter Wäschestücke auf Verunreinigungen befasst sich das Dokument nicht.

Der Erfindung liegt die Aufgabe zugrunde, Verfahren zur Prüfung gewaschener oder gereinigter Wäschestücke zu schaffen, die auf einfache Weise eine umfassende Prüfung der Nutz- bzw. Finishseite gewaschener oder gereinigter Wäschestücke zulassen.

Ein Verfahren zur Lösung der genannten Aufgabe weist die Maßnahmen des Anspruchs 1 auf. Bei diesem Verfahren ist es vorgesehen, dass eine der mindestens einen bildgebenden Einrichtung, beispielsweise einer Kamera oder Sensoren, gegenüberliegende Finishseite des jeweiligen Wäschestücks durchleuchtet wird. Dadurch erfolgt ein Hinterleuchten des jeweiligen Wäschestücks. Durch das Hinterleuchten und/oder Beleuchten der Finishseite scheinen Verunreinigungen dieser Seite des jeweiligen Wäschestücks auf die gegenüberliegende Seite, der die mindestens eine Kamera oder der mindestens eine Sensor zugeordnet sind, durch. Auf der der beleuchteten Seite des Wäschestücks Finishseite werden dadurch Verunreinigungen der anderen, beleuchteten Seite des Wäschestücks für die mindestens eine Kamera oder den mindestens einen Sensor sichtbar.

Es wird eine solche Seite des Wäschestücks beleuchtet, angeleuchtet und/oder hinterleuchtet, bei der es sich um die Nutzseite, die in der Regel von der Finishseite des Wäschestücks gebildet wird, handelt. Dies ist die von den Benutzern später sichtbare Seite. Deswegen ist es insbesondere wichtig, dass diese Finishseite fleckenfrei ist. Die Finishseite befindet sich nach dem Mangeln an der Rückseite des jeweiligen Wäschestücks. Das Beleuchten bzw. Durchleuchten des Wäschestücks an der dem mindestens einen Sensor, Scanner oder der mindestens einen Kamera gegenüberliegenden Finishseite lässt Verunreinigungen an der Finishseite durch das Wäschestück hindurchscheinen, wodurch Verunreinigungen der Finishseite des Wäschestücks von der mindestens einen bildgebenden Einrichtung aufgenommen werden können, vorzugsweise zusammen mit Verunreinigungen und Anhaftungen auf der Vorderseite.

Durch das Hinterleuchten des jeweiligen Wäschestücks scheinen Flecken oder sonstige Verunreinigungen und auch Anhaftungen, wie zum Beispiel Haare, von der angestrahlten Finishseite, deutlich durch das jeweilige Wäschestück hindurch, und zwar auch durch ein mehrlagiges Wäschestück. Dadurch können auch Verunreinigungen der von der Kamera oder Sensoren nicht direkt zugänglichen Finishseite des jeweiligen Wäschestücks von der mindestens einen Kamera oder dergleichen auf der von diesem zugänglichen gegenüberliegenden Seite, insbesondere Vorderseite, des Wäschestücks zuverlässig erfasst werden.

Das erfindungsgemäße Verfahren ermöglicht es, Verunreinigungen und Anhaftungen auf beiden Seiten des Wäschestücks von mindestens einer Kamera oder einem Sensor oder einer sonstigen bildgebenden Einrichtung an nur einer Seite, insbesondere der Vorderseite, des Wäschestücks zu detektieren und auszuwerten. Es werden Verunreinigungen auf der Finishseite an der gegenüberliegenden Seite, insbesondere der Ober- bzw. Vorderseite, sichtbar gemacht, so dass diese auf beiden Seiten des Wäschestücks von einer Kamera oder sonstigen bildgebenden Einrichtungen von nur einer Seite des jeweiligen Wäschestücks festgestellt werden können.

Die Prüfung des Wäschestücks wird im ausgebreiteten Zustand desselben durchgeführt. Dadurch ist eine lückenlose und vollständige, insbesondere vollflächige, Be- und/oder Durchleuchtung des Wäschestücks und dadurch eine vollflächige Prüfung auch der Rückseite des jeweiligen Wäschestücks möglich.

Gemäß der Erfindung findet die Prüfung bei mit der Finishseite ausgebreitet auf mindestens einem Förderer liegenden Wäschestück statt, wobei die auf dem Förderer aufliegende Finishseite vorzugsweise von unten angeleuchtet, hinterleuchtet bzw. beleuchtet wird. Dadurch kommt es zu einem Durchleuchten des Wäschestücks, wodurch Flecken oder sonstige Verunreinigungen einschließlich Anhaftungen auf der Finishseite, die von der Kamera oder dergleichen weggerichtet ist, auch detektiert werden können. Es sind so Flecken auf beiden Seiten des Wäschestücks ermittelbar, obwohl die Flecken auf der stets unten auf dem Förderer liegenden Finishseite von der Kamera, dem Scanner bzw. dem mindestens einen Sensor weggerichtet ist, weil sie auf der Seite des Förderers liegen.

Eine weitere vorteilhafte Ausgestaltungsmöglichkeit des Verfahrens sieht es vor, dass die der mindestens einen Kamera bzw. dem mindestens einen Scanner gegenüberliegende Finishseite des Wäschestücks gleichmäßig und/oder durchgehend über die ganze Breite durchleuchtet wird. Dadurch werden alle Flecken, Fremdkörper und/oder Beschädigungen auch auf der dem Scanner oder der Kamera gegenüberliegenden Seite sichtbar gemacht, so dass auch diese von der mindestens einen Kamera oder dem mindestens einen Scanner sichtbar und so zuverlässig erfassbar sind.

Insbesondere ist es vorgesehen, dass das jeweilige Wäschestück von der der mindestens einen Kamera oder dem mindestens einen Scanner gegenüberliegenden Finishseite derart durchleuchtet wird, dass Flecken oder sonstige Verunreinigungen, Beschädigungen (Löcher) und auch Anhaftungen der Finishseite auf der gegenüberliegenden Seite, auf der sich die mindestens eine Kamera oder dergleichen befinden, sichtbar werden. Auch Flecken oder sonstige Verunreinigungen bzw. Anhaftungen auf der verdeckten Finishseite des Wäschestücks, der keine Kamera oder dergleichen zugeordnet sind oder zugeordnet werden können, weil die Finishseite auf dem Förderer liegt, können so ohne ein Umdrehen des Wäschestücks ermittelt werden.

Eine andere Ausgestaltungsmöglichkeit des Verfahrens sieht es vor, das Wäschestück von einer der Kamera oder Ähnlichem weggerichteten Seite derart zu durchleuchten, dass mindestens Verunreinigungen und Anhaftungen auf der hinterleuchteten bzw. angestrahlten Finishseite auf der zur mindestens einen Kamera weisenden gegenüberliegenden Seite des Wäschestücks sichtbar. Es erscheinen Flecken, Anhaftungen, Beschädigungen und sonstige Beeinträchtigungen beider Seiten des Wäschestücks auf derjenigen Seite, worauf die mindestens eine Kamera oder Ähnliches gerichtet ist.

Eine Weiterbildungsmöglichkeit des Verfahrens sieht es vor, dass beide Seiten des jeweiligen Wäschestücks beleuchtet werden. Es wird also nicht nur die von der Kamera oder dem Sensor weggerichteten Seite des jeweiligen Wäschestücks beleuchtet, sondern auch von der gegenüberliegenden Seite, auf die die Kamera bzw. der Sensor gerichtet sind. Die beidseitige Beleuchtung und/oder Durchleuchtung des Wäschestücks ist besonders vorteilhaft bei einem mehrlagigen Wäschestück. Verunreinigungen, Anhaftungen oder auch Muster des Wäschestücks erscheinen so für die Kamera oder Sensoren kontrastreicher, was die Erkennung von Flecken, Haaren, Mustern oder dergleichen auf beiden Seiten des jeweiligen Wäschestücks vereinfacht und zuverlässiger gestaltet und auch Löcher besser erkennbar macht.

Bevorzugte Ausführungsbeispiele der Erfindung werden nachfolgend anhand der Zeichnung näher erläutert. In dieser zeigen:
- Fig. 1: eine schematische Seitenansicht einer Vorrichtung zur Durchführung des erfindungsgemäßen Verfahrens mit einem Förderer zwischen einer Mangel und einer darauf folgenden Faltmaschine, und
- Fig. 2: eine schematische Seitenansicht einer alternativen Vorrichtung zur Durchführung des erfindungsgemäßen Verfahrens.

Bei den gezeigten Ausführungsbeispielen finden die erfindungsgemäßen Verfahren zwischen einer Mangel 10 und einer Faltmaschine 11 statt. Jedes in den Figuren von der Seite gezeigtes gemangelte Wäschestück 12 wird nach dem Verlassen eines Auslaufbereichs 13 der Mangel 10 von einem Förderer in Behandlungsrichtung 15 zur Faltmaschine 11 transportiert. In der Faltmaschine 11 wird das gemangelte Wäschestück 12 automatisch gefaltet.

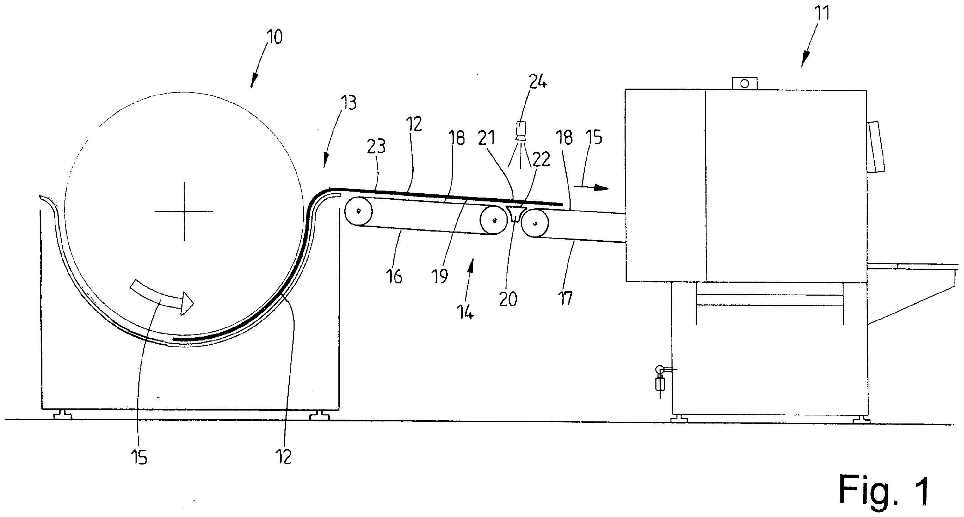

Im Ausführungsbeispiel der Fig. 1 ist der Förderer 14 von zwei aufeinanderfolgenden Förderern gebildet, und zwar einem Auslaufförderer 16 hinter dem Auslaufbereich 13 der Mangel 10 und einem sich daran anschließenden Einlaufförderer 17 vor der Faltmaschine 11. Der Auslaufförderer 16 und der Einlaufförderer 17 sind gleichermaßen als Gurtförderer mit einem umlaufenden Gurttrieb ausgebildet. Der Auslaufförderer 16 und der Einlaufförderer 17 können einen über die gesamte Arbeitsbreite durchgehenden Fördergurt oder auch mehrere nebeneinanderliegende schmale Fördergurte aufweisen. Über die Obertrume 18 des Auslaufförderers 16 und des Einlaufförderers 17 werden gemangelte und dadurch ausgebreitete, vorzugsweise flach ausgestreckte, Wäschestücke 12 ohne Überlappung, vorzugsweise mit Abstand zueinander, aufeinanderfolgend in Behandlungsrichtung 15 zur Faltmaschine 11 transportiert. Bei mehrbahniger Betriebsweise können auch die Wäschestücke 12 in mehreren Bahnen nebeneinanderliegend auf dem Förderer 16, 17 liegen. Gegebenenfalls können die Obertrume 18 perforiert sein, um mit Unterdruck die ausgebreiteten Wäschestücke 12 zumindest bereichsweise auf dem Auslaufförderer 16 bzw. dem Einlaufförderer 17 zu fixieren. Alternativ ist es auch denkbar, den Auslaufförderer 16 und den Einlaufförderer 17 mit netzartigen Gurten zu versehen. Die Wäschestücke 12 liegen dann auf dem netz- oder gitterartigen Obertrum 18 auf. Dadurch wird eine auf dem Förderer 14 aufliegende Unterseite jedes Wäschestücks 12 vom Förderergurt des Förderers 14, insbesondere des Auslaufförderers 16 und des Einlaufförderers 17, nahezu vollständig freigelassen.

Die Wäschestücke 12 verlassen die Mangel 10 mit unten liegender Finishseite 19, weil sich üblicherweise die Finishseite 19 der Wäschestücke 12 an der unteren stillstehenden Mangelmulde bildet. Mangeln anderer Bauart, zum Beispiel Bändermangeln, können die Wäschestücke 12 auch mit obenliegender Finishseite 19 verlassen. Deshalb ist die Erfindung nicht nur auf Wäschestücke 12 mit untenliegender Finishseite 19 beschränkt.

Die Finishseite 19 ist die spätere Nutzseite der Wäschestücke 12, und zwar bei Tischwäsche die Oberseite. Weil die die spätere Oberseite der Wäschestücke 12 bildende Finishseite 19 beim Verlassen der Mangel 10 unten liegt und damit auf dem Obertrum 18 des auf die Mangel 10 folgenden Förderers 14 bzw. Auslaufförderer 16 und Einlaufförderer 17, sind Muster, Löcher und Verunreinigungen, insbesondere Flecken, bei untenliegender Finishseite 19 des Wäschestücks 12 schwer ermittelbar. Bislang musste das jeweilige Wäschestück 12 dazu gewendet werden.

Erfindungsgemäß ist zwischen der Mangel 10 und der Faltmaschine 11 mindestens eine Leuchteinrichtung 20 vorgesehen. Die Leuchteinrichtung 20 ist derart zwischen der Mangel 10 und der Faltmaschine 11 angeordnet, dass sie die Wäschestücke 12 von unten, vorzugsweise ihren untenliegenden Finishseiten 19, her beleuchtet oder anleuchtet, wodurch es zu einem Hinterleuchten und/oder Durchleuchten der Wäschestücke 12 kommt. Dementsprechend ist die Leuchtstärke der Leuchteinrichtung 20 so bemessen, dass Löcher, Muster, Anhaftungen und Verunreinigungen, vor allem Flecken, an der Unterseite des jeweiligen Wäschestücks 12 zur oberen, freiliegenden Seite durchscheinen und dort auch sichtbar sind.

Die Leuchteinrichtung 20 beleuchtet einen quer zur Behandlungsrichtung 15 verlaufenden Querstreifen 21 jedes Wäschestücks 12. Zu diesem Zweck entspricht die Breite der Leuchteinrichtung 20 der Breite des Förderers 14, insbesondere des Auslaufbereichs 16 und des gleich breiten Einlaufbereichs 17.

Im Ausführungsbeispiel der Fig. 1 ist die Leuchteinrichtung 20 derart dem Förderer 14 zugeordnet, dass sie sich zwischen dem Auslaufförderer 16 und dem Einlaufförderer 17 befindet. Dabei überbrückt die Leuchteinrichtung 20 einen Spalt zwischen dem Auslaufförderer 16 und dem Einlaufförderer 17, befindet sich nämlich in einem zwischen den angrenzenden Umlenk- bzw. Antriebstrommeln des Auslaufförderers 16 und des Einlaufförderers 17 liegenden oberen Keilbereich des Förderers 14. Dazu ist an diesen oberen Keilbereich des Förderers 14 der Querschnitt der Leuchteinrichtung 20 angepasst, so dass eine im gezeigten Ausführungsbeispiel ebene Oberseite 22 eines Gehäuses der Leuchteinrichtung 20 sich etwa in der Ebene des Obertrums 18 des Förderers 14, insbesondere des Auslaufförderers 16 und des Einlaufförderers 17, befindet. Im Gehäuse der Leuchteinrichtung 20 können die Leuchtmittel, vorzugsweise LED-Leuchtmittel, untergebracht sein, und zwar so, dass von den Leuchtmitteln ausgehende Strahlen nach oben durch die Oberseite 22 des Gehäuses hindurch gegen die Unterseite des Wäschestücks 12 gerichtet sind. Es ist auch denkbar, Leuchtstreifen mit darauf angeordneten kleinen LED-Leuchtmitteln auf die Oberseite 22 des Gehäuses aufzukleben. Dabei sind die LED-Leuchtmittel wiederum so orientiert, dass die von diesen abgesendeten Lichtstrahlen gegen die Unterseite des Wäschestücks 12 gerichtet und auf die Unterseite, vorzugsweise die Finishseite 19, des Wäschestücks 12 auftreffen.

Der dem Förderer 14 gegenüberliegenden Seite, insbesondere einer Oberseite 23 des Wäschestücks 12, ist eine bildgebende Einrichtung zugeordnet, die im gezeigten Ausführungsbeispiel als mindestens eine Kamera 24 ausgebildet ist. Die Kamera 24 ist mit Abstand über dem Wäschestück 12 ortsfest positioniert. Diese Positionierung der Kamera 24 erfolgt derart, dass die Kamera 24 die Oberseite 23 des Wäschestücks 12 vorzugsweise vollflächig abbildet. Dabei ist die Kamera 24 so ausgebildet, dass sie zumindest den von der Leuchteinrichtung 20 gleichmäßig und vollständig, vor allem lückenlos, hinterleuchteten, angeleuchteten und/oder durchleuchteten Querstreifen 21 des Wäschestücks 12 über die gesamte Breite desselben erfasst und hiervon ein Bild mit den Verunreinigungen, Löchern, Mustern oder dergleichen auf der unteren, durchleuchteten Finishseite 19 und vorzugsweise auch zugleich der Oberseite 23 erzeugt.

Erforderlichenfalls kann auf der Seite der Kamera 24 auch noch mindestens eine Leuchtquelle vorgesehen sein. Diese Leuchtquelle kann gegebenenfalls in die Kamera 24 integriert sein. Die mindestens eine Leuchtquelle auf der Seite der Kamera 24, also der freiliegenden Oberseite des Wäschestücks 12, leuchtet vorzugsweise auch einen Querstreifen 21 des Wäschestücks 12 über die gesamte Breite aus. Auch bei dieser mindestens einen Lichtquelle kann es sich um eine Reihe mehrerer LED-Leuchtmittel handeln. Diese Leuchtmittel erzeugen ein auf die freiliegende Oberseite des Wäschestücks 12 gerichtetes Auflicht. In diesem Fall wird das Wäschestück im Bereich des Querstreifens 21 beidseitig beleuchtet, und zwar von der freiliegenden Oberseite mit einem Auflicht und auf der gegenüberliegenden, dem Förderer 14 zugewandten Unterseite mit Licht hinter- und durchleuchtet. Die doppelseitige Beleuchtung, insbesondere das An- und Durchleuchten des jeweiligen Wäschestücks 12, erleichtert und verbessert die Erkennung von Beeinträchtigungen des jeweiligen Wäschestücks 12, insbesondere Flecken, Löcher oder dergleichen. Die beidseitige Be- und Durchleuchtung des jeweiligen Wäschestücks 12 lässt auch eine bessere und zuverlässige Erkennung von Mustern des Wäschestücks 12 oder Fremdkörpern auf demselben, beispielsweise Haare, zu.

Es ist denkbar, mehrere Kameras 24 oder sonstige bildgebende Einrichtungen vorzusehen, insbesondere in einer quer zur Behandlungsrichtung 15 verlaufenden Reihe. Alternativ könnten an der Stelle der Kamera 24 auch lichtsensitive Sensoren vorgesehen sein, vorzugsweise mehrere Sensoren nach Art eines Scanners in einer quer zur Behandlungsrichtung 15 verlaufenden Reihe mit gleichmäßigem Abstand nebeneinander angeordnet sein, so dass sie die gesamte Breite des Wäschestücks 12 bzw. die Breite mehrerer nebeneinanderliegender Wäschestücke 12, lückenlos abscannen.

Die mindestens eine Kamera 24 ist bevorzugt mit einer selbst-fokussierenden Optik versehen. Diese ermöglicht eine automatische Scharfeinstellung. Dadurch können zum Beispiel auch Beeinträchtigungen oder Fremdkörper an einem Wäschestück 12 erkannt werden, das im Bereich der mindestens einen Kamera 24 nicht flach auf dem Förderer 14 aufliegt, sondern etwas aufgewölbt ist. Außerdem ist die automatische Scharfeinstellung der mindestens einen Kamera 24 in der Lage, erforderlichenfalls eine Verstellung derart vorzunehmen, dass zum Beispiel Flecken auf der zur Kamera 24 weisenden Oberseite des Wäschestücks 12 und/oder von der von unten angeleuchteten Unterseite des Wäschestücks 12 durchscheinende Flecken oder dergleichen scharf erscheinen. Die automatische Einstellung der Optik der mindestens einen Kamera 24 kann auch in Abhängigkeit davon erfolgen, was sie momentan aufnimmt, beispielsweise einen Fleck, ein Loch, ein Muster oder dergleichen.

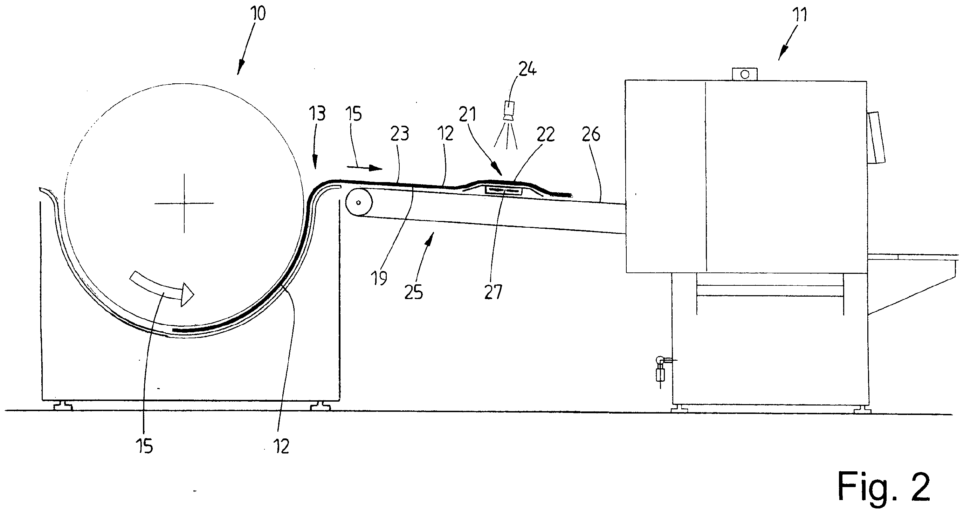

Die Fig. 2 zeigt ein alternatives Ausführungsbeispiel mit einem einzigen durchgehenden Förderer 25 zwischen der Mangel 10 und der Faltmaschine 11. Dicht über einem Obertrum 26 dieses Förderers 25 ist eine über die gesamte Breite des Förderers 25 quer zur Behandlungsrichtung 15 durchgehende Leuchteinrichtung 27 angeordnet. Das Obertrum 26 ist unter der Leuchteinrichtung 27 hinweggeführt, wobei entsprechende Leitbleche im Bereich der Leuchteinrichtung 27 das Wäschestück 12 vom Obertrum 26 ablenken, insbesondere abheben, so dass das Wäschestück 12 im Bereich der Leuchteinrichtung 27 über denselben hinweg transportiert wird. Dadurch wird die Leuchteinrichtung 27 nicht vom Obertrum 26 überdeckt, so dass die Leuchteinrichtung 27 direkt mit der Unterseite, insbesondere Finishseite 19, des Wäschestücks 12 in Kontakt kommt und dieses von unten anleuchten und/oder durchleuchten kann, so dass Flecken, Löcher oder dergleichen an bzw. in der Unterseite des Wäschestücks 12 zur Oberseite desselben durchscheinen und so auch von der mindestens einen der Oberseite des Wäschestücks 12 zugeordneten Kamera 24 erfassbar sind. Falls das Obertrum 26 über die Leuchteinrichtung 27 hinweggeführt wird, ist das Obertrum 26 des Förderers 14 entweder lichtdurchlässig ausgebildet oder es ist netz- oder gitterartig ausgebildet und dadurch nahezu vollständig lichtdurchlässig.

Abweichend vom Ausführungsbeispiel der Fig. 2 kann eine flach ausgebildete Leuchteinrichtung 27 auch zwischen den Trumen des Förderers 25 angeordnet sein, so dass die mit der Unterseite, insbesondere Finishseite 19, des jeweiligen Wäschestücks 12 in Kontakt kommende Oberseite 22 der Leuchteinrichtung 27 in der Hauptebene des Obertrums 26 (vor und hinter der Leuchteinrichtung 27) liegt und dadurch die Leuchteinrichtung 27 im Gegensatz zum Ausführungsbeispiel der Fig. 2 keine Erhöhung über dem Obertrum 26 des Förderers 25 bildet.

Die Leuchteinrichtung 27 kann wie die im vorangehenden Ausführungsbeispiel näher beschriebene Leuchteinrichtung 20 ausgebildet sein. Zur Vermeidung von Wiederholungen wird auf die Beschreibung der Leuchteinrichtung 20 Bezug genommen.

Nachfolgend wird das erfindungsgemäße Verfahren anhand der Vorrichtung der Fig. 1 näher erläutert:
Mit dem erfindungsgemäßen Verfahren ist es möglich, Verunreinigungen, insbesondere Flecken, aber auch Löcher, Anhaftungen, zum Beispiel Haare, und dergleichen, auf der Oberseite 23 und der Unterseite, insbesondere der Finishseite 19, von Wäschestücken 12 gleichzeitig zu ermitteln, ohne die Wäschestücke 12 wenden zu müssen. Die Ermittlung von Verunreinigungen, vor allem Flecken und Anhaftungen, und auch Löcher erfolgt kontinuierlich auf dem Weg des Wäschestücks 12 von der Mangel 10 zur Faltmaschine 11. Dadurch können die Verunreinigungen, Löcher und Anhaftungen am vorzugsweise flach und ausgestreift auf dem Obertrum 18 des Förderers 14 zwischen der Mangel 10 und der Faltmaschine 11 liegenden gemangelten Wäschestücks 12 detektiert werden.

Von der mindestens einen Kamera 24 oder einer anderen bildgebenden Einrichtung wird beim Vorbeilaufen kontinuierlich nach und nach jeweils ein Querstreifen 21 des Wäschestücks 12 mit durchscheinenden unteren Anhaftungen und Verunreinigungen und auch oberen Verunreinigungen und Anhaftungen sowie Löchern aufgenommen. Dabei scannt die Kamera 24 nach und nach die gesamte Oberseite 23 des Wäschestücks vollflächig und erzeugt hiervon mindestens ein Bild.

Verunreinigungen, insbesondere Flecken, auf der Oberseite 23 des jeweiligen Wäschestücks 12 werden von der Kamera 24 direkt erfasst. Verunreinigungen, insbesondere Flecken, auf zum Obertrum 18 des Förderers 14 weisenden oder aufliegenden Unterseite bzw. Finishseite 19 des jeweiligen Wäschestücks 12 werden erfindungsgemäß auch von der Kamera 24 von der Oberseite 23 her ermittelt, indem von der mindestens einen Leuchteinrichtung 20 die Unterseite, insbesondere Finishseite 19, des jeweiligen Wäschestücks beleuchtet oder angestrahlt wird. Dies erfolgt derart, dass das Wäschestück 12 von der Finishseite 19 her durchleuchtet wird, wodurch Verunreinigungen, insbesondere Flecken, der Unterseite bzw. Finishseite 19 des jeweiligen Wäschestücks 12 durch das Wäschestück 12 hindurchscheinen und dadurch auch an der Oberseite 23 des Wäschestücks 12 von der Kamera 24 ermittelbar, vor allem abbildbar, sind. Auf diese Weise kann die mindestens eine nur einer Seite des Wäschestücks 12 zugeordnete Kamera 24 oder eine sonstige bildgebende Einrichtung nicht nur Löcher oder sonstige Beschädigungen, sondern auch Verunreinigungen sowohl auf der Oberseite 23 als auch auf der untenliegenden Seite, zum Beispiel der Finishseite 19, gleichzeitig ermitteln. Zu diesem Zweck sind die Leuchtmittel oder Leuchtquellen der Leuchteinrichtung 20 ausgebildet, um genügend Licht zu erzeugen, dass Flecken oder sonstige Verunreinigungen an der Unterseite bzw. Finishseite 19 des jeweiligen Wäschestücks 12 durch dasselbe hindurchscheinen und auf der Oberseite 23 für die mindestens eine Kamera 24 oder eine sonstige bildgebende Einrichtung sichtbar werden.

In der vorstehend beschriebenen Weise läuft prinzipiell genauso das Verfahren bei der Vorrichtung der Fig. 2 ab.

Weiterhin kann es vorgesehen sein, von der mindestens einen Kamera, Sensoren oder dergleichen das Wäschestück gleichzeitig zu identifizieren, so dass ermittelbar ist, um welches Wäschestück es sich handelt. Das ermöglicht eine Feststellung, ob am gleichen Wäschestück mehrfach die gleiche Beeinträchtigung, beispielsweise der gleiche Fleck oder das gleiche Loch, erkannt worden ist. Wird zum Beispiel ein gleicher Fleck mehrfach detektiert, deutet das darauf hin, dass dieser Fleck nicht entfernbar ist. Ein solches Wäschestück kann dann aussortiert werden und braucht nicht wiederholt gewaschen zu werden. Festgestellt werden kann auch, wie sich ein bestimmter Fleck beim wiederholten Waschen oder einer sonstigen Behandlung verändert. Wird keine Veränderung festgestellt, deutet das auf eine Beeinträchtigung, beispielsweise einen Fleck, hin, der nicht entfernbar ist. Wenn die Beeinträchtigung, beispielsweise der Fleck, aber bei wiederholtem Waschen abnimmt, kann durch ein mindestens einmal wiederholtes Waschen versucht werden, den Fleck weiter oder ganz zu entfernen.

## Patentansprüche

1. Verfahren zur Prüfung gewaschener oder gereinigter Wäschestücke (12), wobei Verunreinigungen, insbesondere Flecken, durch mindestens eine auf eine Seite des jeweiligen Wäschestücks (12) gerichtete Kamera (24) oder mindestens einen Sensor erfasst werden, indem eine einer solchen Seite, der die mindestens eine Kamera (24) bzw. der mindestens eine Sensor zugeordnet sind, gegenüberliegende Seite des Wäschestücks (12) durchleuchtet wird, **dadurch gekennzeichnet, dass** eine solche Seite des jeweiligen Wäschestücks (12) durchleuchtet wird, bei der es sich um eine Finishseite (19) des Wäschestücks (12) handelt, die sich an einer unteren, stillstehenden Mangelmulde einer Mangel (10) bildet, so dass sie beim die Mangel (10) verlassenden Wäschestück (12) unten liegt und mit der Finishseite (19) das die Mangel (10) verlassende Wäschestück (12) im ausgebreiteten Zustand auf einem Obertrum (18) eines auf die Mangel (10) folgenden Förderers (14) liegt, wobei auf dem Förderer (14) liegende Finishseite (19) von unten durchleuchtet wird und dabei Verunreinigungen, insbesondere Flecken, auf einer vom Förderer (14) weggerichteten Oberseite (23) sowie der Finishseite (19) des jeweiligen Wäschestücks (12) gleichzeitig ermittelt werden.

2. Verfahren nach Anspruch 1, **dadurch gekennzeichnet, dass** Verunreinigungen, insbesondere Flecken, an der durchleuchteten Seite des Wäschestücks (12) durch dasselbe hindurchscheinen und dadurch auf der gegenüberliegende Seite des Wäschestücks (12), der die mindestens eine Kamera (24), der mindestens eine Sensor oder eine sonstige bildgebende Einrichtung zugeordnet ist, sichtbar werden bzw. sichtbar gemacht werden.

3. Verfahren nach Anspruch 1, **dadurch gekennzeichnet, dass** die der mindestens einen Kamera (24) bzw. dem mindestens einen Sensor gegenüberliegende Finishseite (19) des Wäschestücks (12) gleichmäßig über die gesamte Breite des jeweiligen Wäschestücks (12) durchgehend, insbesondere im Bereich eines Querstreifens (21) des jeweiligen Wäschestücks (12), durchleuchtet wird, vorzugsweise kontinuierlich beim Weitertransport des jeweiligen Wäschestücks (12) vom Förderer (14, 25) in Behandlungsrichtung (15).

4. Verfahren nach einem der vorhergehenden Ansprüche, **dadurch gekennzeichnet, dass** die Wäschestücke (12) von der der mindestens einen Kamera (24) bzw. dem mindestens einen Sensor gegenüberliegenden Finishseite (19) derart beleuchtet werden, dass sie durchleuchtet werden und/oder Verunreinigungen, insbesondere Flecken, auf der Finishseite (19) durch die Wäschestücke (12) durchscheinen und so auch Verunreinigungen bzw. Flecken auf der Finishseite (19) der Wäschestücke (12) auf der gegenüberliegenden Seite, der die mindestens eine Kamera (24) bzw. der mindestens eine Sensor zugeordnet ist, sichtbar sind.

5. Verfahren nach einem der vorhergehenden Ansprüche, **dadurch gekennzeichnet, dass** die Wäschestücke (12) von ihrer der mindestens einen Kamera (24) bzw. dem mindestens einen Sensor weggerichteten Seite derart beleuchtet werden, dass mindestens Flecken auf der beleuchteten Seite an der zur mindestens einen Kamera (24) und/oder dem mindestens einen Sensor weisenden gegenüberliegenden Seite der Wäschestücke (12) für die zumindest eine Kamera (24) bzw. den mindestens einen Sensor sichtbar werden, indem sie durch die Wäschestücke (12) hindurchscheinen.

## Claims

1. Method for examining washed or cleaned items of laundry (12), wherein contaminants, in particular stains, are detected by at least one camera (24) directed onto one side of the respective item of laundry (12) or by at least one sensor, in that a side of the item of laundry (12) lying opposite such a side associated with the at least one camera (24) or the at least one sensor is transilluminated, **characterized in that** such a side of the respective item of laundry (12) is transilluminated which is the finish side (19) of the item of laundry (12), that forms on a lower, stationary mangle trough such that it lies at the bottom of the item of laundry as it leaves the mangle (10) and the item of laundry (12) leaving the mangle (10) lies in a spread-out state with the finish side (19) on a top strand (18) of a conveyor (14) downstream of the mangle (10), with the finish side (19) lying on the conveyor (14) being transilluminated from below, as a result of which contaminants, in particular stains, can be simultaneously identified on a top side (23) directed away from the conveyor (14) as well as on the finish side (19) of the respective item of laundry (12).

2. Method according to Claim 1, **characterized in that** contaminants, in particular stains, on the transilluminated side of the item of laundry (12) shine through same and thereby become visible or are made visible on the opposite side of the item of laundry (12) which is associated with the at least one camera (24), the at least one sensor, or any other imaging apparatus.

3. Method according to Claim 1, **characterized in that** the finish side (19) of the item of laundry (12) lying opposite the at least one camera (24) or the at least one sensor is transilluminated continuously in a uniform manner over the entire width of the respective item of laundry (12), in particular in the region of a transverse strip (21) of the respective item of laundry (12), preferably continuously during the onward transport of the respective item of laundry (12) in the treatment direction (15) by way of the conveyor (14, 25).

4. Method according to one of the preceding Claims, **characterized in that** the items of laundry (12) are illuminated from the finish side (19) lying opposite the at least one camera (24) or the at least one sensor in such a way that they are transilluminated and/or contaminants, in particular stains, on the finish side (19) shine through the items of laundry (12) and hence contaminants or stains on the finish side (19) of the items of laundry (12) are also visible on the opposite side which is associated with the at least one camera (24) or the at least one sensor.

5. Method according to one of the preceding Claims, **characterized in that** the items of laundry (12) are illuminated from the side thereof directed away from the at least one camera (24) or the at least one sensor in such a way that at least stains on the illuminated side become visible to the at least one camera (24) or the at least one sensor on the other side of the items of laundry (12) pointing to the at least one camera (24) or the at least one sensor by virtue of shining through the items of laundry (12).

## Revendications

1. Procédé, destiné à vérifier des pièces de linge (12) lavées ou nettoyées, des salissures, notamment des taches, étant détectées par au moins une caméra (24) dirigée sur un côté de la pièce de linge (12) en question ou au moins un capteur, en ce qu'un côté de la pièce de linge (12) opposé à un tel côté auquel est associé(e) l'au moins une caméra (24) ou l'au moins un capteur est irradié à cœur, **caractérisé en ce qu'**un côté de la pièce de linge (12) en question est irradié à cœur, qui est un côté de finition (19) de la pièce de linge (12), qui se forme sur une auge de calandre inférieure, immobile d'une calandre (10), de sorte à se situer en bas lorsque la pièce de linge (12) quitte la calandre (10) et que par le côté de finition (19), la pièce de linge (12) quittant la calandre (10) se situe à l'état déployé sur un brin supérieur (18) d'un convoyeur (14) suivant la calandre (10), le côté de finition (19) situé sur le convoyeur (14) étant irradié à cœur par le dessous et à cet effet des salissures, notamment des taches, étant déterminées simultanément sur un côté supérieur (23) opposé au convoyeur (14), ainsi que sur le côté de finition (19) de la pièce de linge (12) en question.

2. Procédé selon la revendication 1, **caractérisé en ce que** des salissures, notamment des taches, sur le côté irradié à cœur de la pièce de linge (12) transparaissent à travers celle-ci et deviennent ainsi visibles ou sont rendues visibles sur le côté opposé de la pièce de linge (12) auquel est associé(e) l'au moins une caméra (24), l'au moins un capteur ou un quelconque autre système d'imagerie.

3. Procédé selon la revendication 1, **caractérisé en ce que** le côté de finition (19) de la pièce de linge (12) opposé à l'au moins une caméra (24) ou à l'au moins un capteur est irradié à cœur régulièrement sur toute la largeur de la pièce de linge (12) en question, constamment, notamment dans la zone d'un ruban transversal (21) de la pièce de linge (12) en question, de préférence en continu, lors du transport ultérieur de la pièce de linge (12) en question par le convoyeur (14, 25), dans la direction de traitement (15).

4. Procédé selon l'une quelconque des revendications précédentes, **caractérisé en ce que** les pièces de linge (12) sont irradiées à partir du côté de finition (19) opposé à l'au moins une caméra (24) ou à l'au moins un capteur, de sorte à être irradiées à cœur et/ou que des salissures, notamment des taches, transparaissent sur le côté de finition (19) à travers les pièces de linge (12) et qu'ainsi également des salissures ou des taches sur le côté de finition (19) des pièces de linge (12) soient visibles sur le côté opposé auquel l'au moins une caméra (24) ou l'au moins un capteur est associé(e) .

5. Procédé selon l'une quelconque des revendications précédentes, **caractérisé en ce que** les pièces de linge (12) sont irradiées à partir de leur côté opposé à l'au moins une caméra (24) ou à l'au moins un capteur, de telle sorte qu'au moins des taches sur le côté irradié deviennent visibles sur le côté opposé des pièces de linge (12) dirigée vers l'au moins une caméra (24) et/ou vers l'au moins un capteur pour l'au moins une caméra (24) ou pour l'au moins un capteur, **en ce qu'**elles transparaissent à travers les pièces de linge (12).
